# EUROPÄISCHE PATENTSCHRIFT

(11) **EP 3 531 156 B1**
(45) Veröffentlichungstag und Bekanntmachung des Hinweises auf die Patenterteilung: **27.03.2024**
(21) Anmeldenummer: 18157877.4
(22) Anmeldetag: 21.02.2018
(51) Int. Cl.: G01R 33/36, G01R 33/565, G01R 33/34, G01R 33/422

(54) **EINSTELLEN EINER FELDVERTEILUNG EINER ANTENNENANORDNUNG EINER MAGNETRESONANZANLAGE**
ADJUSTING A FIELD DISTRIBUTION OF AN ANTENNA ASSEMBLY OF A MAGNETIC RESONANCE SYSTEM
RÉGLAGE D'UNE DISTRIBUTION DE CHAMP D'UN DISPOSITIF D'ANTENNE D'UNE INSTALLATION DE RÉSONANCE MAGNÉTIQUE

(43) Veröffentlichungstag der Anmeldung: 28.08.2019
(73) Patentinhaber: Siemens Healthineers AG, 91301 Forchheim (DE)
(72) Erfinder: Lazar, Razvan, 91056 Erlangen (DE); Nistler, Jürgen, 91056 Erlangen (DE)
(74) Vertreter: Siemens Healthineers Patent Attorneys

(56) Entgegenhaltungen:
- WO-A1-2007/124246
- DE-A1-102006 018 158
- DE-A1-102011 089 448
- DE-B3-102012 207 722
- US-A- 5 081 418
- US-A1- 2009 128 150
- US-A1- 2013 021 033
- US-A1- 2016 363 640
- US-A1- 2018 003 787

## Beschreibung

Die vorliegende Erfindung betrifft ein Verfahren zum Einstellen einer Feldverteilung einer Antennenanordnung einer Magnetresonanzanlage. Die vorliegende Erfindung betrifft insbesondere ein Verfahren zum Kompensieren mechanischer Toleranzen einer Anordnung einer Hochfrequenzantenne in einem Hochfrequenzschirm einer Magnetresonanzanlage. Die vorliegende Erfindung betrifft weiterhin eine Magnetresonanzanlage mit einer Antennenanordnung, ein Computerprogrammprodukt und einen elektronisch lesbaren Datenträger, welche zur Realisierung des Verfahrens beitragen.

In Magnetresonanzanlagen (MR-Anlagen) können Antennenanordnungen verwendet werden, um ein Hochfrequenzfeld, ein sogenanntes B1-Feld, zu erzeugen. Derartige Antennenanordnungen können beispielsweise eine zylindrische oder röhrenförmige Struktur aufweisen, welche rund um eine Patientenaufnahmeöffnung der Magnetresonanzanlage angeordnet ist. Beispiele von Antennenanordnungen, die oftmals auch Ganzkörperspulen (engl. body coil) genannt werden, sind in den Druckschriften US 8362775 B2, US 6781378 B2, US 8072218 B2 und US 20170016969 A1 beschrieben.

Die DE 10 2011 089448 A1 betrifft eine Brustspule für eine Magnetresonanztomographieeinrichtung zur Erzeugung von Magnetresonanzaufnahmen weiblicher Brüste, mit einem Spulengehäuse mit einer Brustausnehmung zur Aufnahme einer Brust und mit einer Anzahl von Spulenelementen. Zumindest eines der Spulenelemente bildet ein HF-Korrekturspulenelement und weist hierzu eine Schaltungsanordnung auf, um das HF-Korrekturspulenelement zwischen einem HF-Korrektur-Betriebszustand und einem weiteren Betriebszustand umzuschalten. Dabei ist das HF-Korrekturspulenelement so ausgebildet, dass es im HF-Korrektur-Betriebszustand passiv mit einem von einer Sendeantennenanordnung der Magnetresonanztomographieeinrichtung ausgesendeten B₁-Feld mitschwingt und die lokale B₁-Feldverteilung während einer Magnetresonanzaufnahme beeinflusst.

Die US 2013/021033 A1 betrifft ein System zum Einstellen eines Funkfrequenz- (RF) Magnetfelds in einer MR Bildgebungseinheit unter Verwendung einer RF Sendespule zum Erzeugen eines Funkfrequenz-(RF) Magnetfelds und mehrerer RF Empfängerspulen zum Empfangen von RF Signalen für eine Magnetresonanz-(MR) Bilddatenerfassung. Eine RF Sendespule erzeugt ein RF Magnetfeld. Eine RF Empfängerspule empfängt ein RF Signal für eine MR Bilddatenerfassung und koppelt ein Magnetfeld von der RF Empfängerspule auf die RF Sendespule zum anpassenden Ändern des von der RF Sendespule erzeugten RF Magnetfelds, um eine Inhomogenität in dem von der RF Sendespule erzeugten RF Magnetfeld als Antwort auf einen Anwenden eines RF Pulses auf die RF Sendespule zu verringern.

Die WO 2007/124246A1 betrifft eine Magnetresonanzanlage mit einer Hochgeschwindigkeitsspulenbetriebsartumschaltung. Ein Hybridschaltkreis zum zusammenarbeitenden Koppeln eines Funkfrequenzsteuersignals mit einer Quadraturspule ist in mindestens einer von mindestens zwei Spulenbetriebsarten konfigurierbar. Eine zeitliche Abfolge der mindestens zwei Spulenbetriebsarten kann bestimmt und verwendet werden, um eine B1 Inhomogenität zu kompensieren.

Die US 5 081 418 A betrifft ein Verfahren zum Einstellen einer Hochfrequenzspule für eine Verwendung bei MR-Bildgebungsanwendungen. Bei dem Verfahren werden Shunts mit einer Reaktanz parallel zu einem Schalter verwendet, um Abschnitte der Spule mit Masse zu verbinden, wodurch ein Resonanzbetrieb der Spule zu einer bekannten Ausrichtung begrenzt wird.

Alternativ zu den o.g. Ganzkörperspulen kann eine derartige Antennenanordnung auch in Verbindung mit einer Lokalspule vorgesehen sein. Die Antennenanordnung kann eine eigentliche Antenne, beispielsweise eine sogenannte Vogelkäfigantenne (Birdcage Antenna) oder einen transversal elektromagnetischen Resonator (TEM-Antenne), und einen Hochfrequenzschirm (HF-Schirm) umfassen, um eine definierte Umgebung für die Felderzeugung bereitzustellen. Für eine optimale Arbeitsweise der Antennenanordnung ist das Zusammenspiel von Antenne und HF-Schirm entscheidend, um beispielsweise eine symmetrische oder homogene Feldverteilung zu erreichen. Unter anderem ist dabei der Abstand zwischen Antenne und HF-Schirm möglichst exakt einzuhalten. Praktische Realisierungen derartiger Antennenanordnungen beruhen jedoch auf einer mechanischen Trennung von Antenne und HF-Schirm, wodurch Einstellelemente erforderlich sind, um die vorhandenen Toleranzen zu kompensieren.

Neben mechanischen Einstellelementen können einstellbare Kondensatoren, sogenannte Trimmkondensatoren, vorgesehen werden, um diese mechanischen Toleranzen auszugleichen und die Feldverteilung zu verbessern, beispielsweise zu homogenisieren oder eine Feldsymmetrie erhöhen. Derartige Einstellenarbeiten sind jedoch sehr zeit- und somit kostenintensiv. Ferner kann die homogene oder symmetrische Feldverteilung des von der Antennenanordnung abgegebenen B1-Feldes auch im Betrieb der Magnetresonanzanlage durch beispielsweise den zu untersuchenden Patienten beeinflusst werden.

Aufgabe der vorliegenden Erfindung ist es daher, eine Feldverteilung einer Antennenanordnung einer Magnetresonanzanlage beispielsweise unter den zuvor beschriebenen Bedingungen zu verbessern.

Erfindungsgemäß wird diese Aufgabe durch ein Verfahren zum Einstellen einer Feldverteilung einer Antennenanordnung einer Magnetresonanzanlage, eine Magnetresonanzanlage, ein Computerprogrammprodukt und einen elektronisch lesbaren Datenträger gemäß der unabhängigen Ansprüche gelöst. Die abhängigen Ansprüche definieren Ausführungsformen der Erfindung.

Gemäß der vorliegenden Erfindung wird ein Verfahren zum Einstellen einer Feldverteilung einer Antennenanordnung einer Magnetresonanzanlage gemäß Anspruch 1 bereitgestellt. Die Antennenanordnung ist eine zylinderförmige Antennenanordnung zur Erzeugung von Hochfrequenzsignalen, die einen zylinderförmigen Hochfrequenzschirm rund um die Antennenanordnung aufweist. Eine Symmetrie der Feldverteilung ist durch eine Positionsungenauigkeit der Antennenanordnung in der Magnetresonanzanlage beeinträchtigt. Eine Symmetrie der Feldverteilung ist durch eine Positionsungenauigkeit der Antennenanordnung in Bezug auf den Hochfrequenzschirm (HF-Schirm) beeinträchtigt. Eine Beeinträchtigung der Symmetrie der Feldverteilung kann beispielsweise eine Beeinträchtigung der Homogenität der Feldverteilung im Inneren der Antennenanordnung umfassen. Die Antennenanordnung umfasst mehrere Antennenschwingkreiselemente und mehrere Schaltelemente. Jedem Antennenschwingkreiselement der mehreren Antennenschwingkreiselemente ist ein jeweiliges Schaltelement der mehreren Schaltelemente zugeordnet. Ein jeweiliges Schaltelement ist ausgestaltet, dass zugeordnete Antennenschwingkreiselement in Abhängigkeit von einem Schaltzustand des Schaltelements mit der Antennenanordnung funktionsfähig zu koppeln oder von der Antennenanordnung zu entkoppeln. Die Kombination aus Antennenschwingkreiselement und zugeordnetem Schaltelement kann somit als ein schaltbares Antennenschwingkreiselement betrachtet werden.

Die Antennenanordnung kann beispielsweise eine Antenne mit einer Vogelkäfigstruktur (englisch: Birdcage structure) umfassen. Eine Vogelkäfigstruktur besteht aus einer Anzahl von auf einer zylinderartigen Oberfläche angeordneten, äquidistanten, parallel verlaufenden Antennen-Längsstäben. Diese Längsstäbe sind endseitig jeweils durch Antennen-Endringe hochfrequenzmäßig untereinander verbunden. Eine hochfrequenzmäßige Verbindung bedeutet in diesem Zusammenhang, dass nicht zwingend eine galvanische Verbindung, sondern lediglich eine für Hochfrequenzströme transparente Verbindung besteht. Die schaltbaren Antennenschwingkreiselemente können beispielsweise zwischen galvanisch getrennten Abschnitten der Endringe angeordnet sein. Alternativ können die schaltbaren Antennenschwingkreiselemente beispielsweise parallel zu einem der Längsstäbe angeordnet sein.

Bei dem Verfahren wird eine Symmetrieinformation der Feldverteilung in der Antennenanordnung gemessen. Insbesondere kann beispielsweise eine Symmetrieinformation der Feldverteilung im Inneren der Antennenanordnung gemessen werden. Im Fall einer Antennenanordnung mit Vogelkäfigstruktur kann beispielsweise eine Symmetrieinformation der Feldverteilung innerhalb der zylinderförmigen Oberfläche, welche von den Längsstäben begrenzt wird, gemessen werden. Anhand der gemessenen Symmetrieinformation werden die mehreren Schaltelemente automatisch derart eingestellt, dass eine Symmetrie der Feldverteilung im Inneren der Antennenanordnung erhöht wird.

Durch die Verwendung von Schaltelementen kann ein Einstellen beziehungsweise Abgleichen der Antennenanordnung auf einfache Art und Weise automatisiert werden. Eine automatische Steuerung kann beispielsweise verschiedene Schaltkombinationen für die mehreren Schaltelemente mithilfe eines geeigneten Verfahrens ansteuern und so die Feldverteilung optimieren. Beispielsweise kann ein Gradientenabstiegsverfahren dazu verwendet werden oder es können viele oder alle möglichen Schaltkombinationen ausprobiert werden und so die bestmögliche Feldverteilung ermittelt werden. Durch die schaltbaren Antennenschwingkreiselemente können somit mechanische Toleranzen, die sich aus dem Zusammenspiel zwischen Antenne und Hochfrequenzschirm ergeben, kompensiert werden. Ebenso kann im Betrieb der Magnetresonanzanlage eine Rückwirkung eines Patienten auf die Feldverteilung innerhalb der Antennenanordnung kompensiert werden.

Wie zuvor beschrieben, umfasst die Positionsungenauigkeit der Antennenanordnung erfindungsgemäß eine Positionsungenauigkeit einer Anordnung der Antennenanordnung in Bezug auf einen Hochfrequenzschirm.

Diese Positionsungenauigkeit der Anordnung der Antennenanordnung in Bezug auf den Hochfrequenzschirm kann die Symmetrie der Feldverteilung im Inneren der Antennenanordnung beeinträchtigen. Durch das Einstellen der mehreren Schaltelemente derart, dass eine Symmetrie der Feldverteilung im Inneren der Antennenanordnung erhöht wird, werden Auswirkungen der Posierfindungsgemäß zumindest teilweise kompensiert.

Bei einem Ausführungsbeispiel umfasst das Messen der Symmetrieinformation ein Messen einer Resonanzfrequenz der Antennenanordnung. Resonanzfrequenzen und deren Entkopplung können von Symmetrieeigenschaften der Feldverteilung in der Antennenanordnung abhängen. Die mehreren Schaltelemente werden anhand der Resonanzfrequenz derart eingestellt, dass eine Symmetrie der Feldverteilung im Inneren der Antennenanordnung erhöht wird, beispielsweise die mehreren Schaltelemente so eingestellt werden, dass eine oder mehrere bestimmte (gewünschte) Resonanzfrequenzen und deren Entkopplung auftreten. Die Resonanzfrequenz der Antennenanordnung kann beispielsweise beim Einspeisen eines Hochfrequenzsignals in die Antennenanordnung bestimmt werden und ist somit auf einfache Art und Weise als Maß für die Symmetrie oder Homogenität der Feldverteilung verfügbar.

Bei einem weiteren Ausführungsbeispiel umfasst das Messen der Symmetrieinformation ein Messen eines B1-Feldes in der Antennenanordnung. Die mehreren Schaltelemente werden anhand der B1-Feld-Messung derart eingestellt, dass eine Symmetrie der Feldverteilung im Inneren der Antennenanordnung erhöht wird. Die Messung des B1-Feldes kann beispielsweise mithilfe von entsprechenden Sensoren an geeigneten Stellen im Inneren der Antennenanordnung durchgeführt werden. Dadurch kann beispielsweise die Homogenität oder Symmetrie in verschiedenen besonders relevanten Bereichen sichergestellt werden.

Bei noch einem weiteren Ausführungsbeispiel umfasst das Messen der Symmetrieinformation ein Messen einer Stromverteilung in der Antennenanordnung. Die mehreren Schaltelemente werden anhand der Stromverteilung derart eingestellt, dass eine Symmetrie der Feldverteilung im Inneren der Antennenanordnung erhöht wird. Die Stromverteilung in der Antennenanordnung kann beispielsweise mit geeigneten Stromsensoren, sogenannten Pick-up-Spulen durchgeführt werden. Die Stromsensoren können beispielsweise den Schaltelementen zugeordnet sein. Alternativ oder zusätzlich können die Stromsensoren einzelnen Elementen der Endringe oder der Längsstäbe einer Antenne mit Vogelkäfigstruktur zugeordnet sein. Weiterhin können die Stromsensoren in Bereichen einer galvanischen Trennung zwischen einzelnen Elementen der Endringe angeordnet sein.

Die Stromverteilung stellt einen Indikator für die Homogenität bzw. Symmetrie der Feldverteilung von dem von der Antennenanordnung erzeugten Hochfrequenzfeld dar und ist somit zum Steuern der Schaltelemente geeignet.

Ein Antennenschwingkreiselement der mehreren Antennenschwingkreiselemente kann beispielsweise einen Kondensator umfassen. Der Kondensator wird mittels des zugeordneten Schaltelements mit der Antennenanordnung gekoppelt, um eine Kapazität der Antennenanordnung zur Erhöhung der Symmetrie oder Homogenität der Feldverteilung in der Antennenanordnung zu verändern. Beispielsweise kann ein Kondensator in Reihe mit dem zugeordneten Schaltelement zwischen zwei galvanisch getrennten Elementen von einem Endring angeordnet werden, um in Abhängigkeit von dem Schaltzustand des Schaltelements die kapazitive Kopplung zwischen den galvanisch getrennten Elementen des Endrings zu erhöhen. In Abhängigkeit von der Asymmetrie oder Inhomogenität der Feldverteilung kann eine derartige Erhöhung der kapazitiven Kopplung die Symmetrie oder Homogenität der Feldverteilung verbessern.

Alternativ oder zusätzlich kann ein Antennenschwingkreiselement der mehreren Antennenschwingkreiselemente beispielsweise ein induktives Element umfassen. Das induktive Element wird mittels des zugeordneten Schaltelements mit der Antennenanordnung gekoppelt, um eine Induktivität der Antennenanordnung zur Erhöhung der Symmetrie oder Homogenität der Feldverteilung zu verändern. Zum Beispiel kann eine Reihenschaltung bestehend aus dem induktiven Element und dem zugeordneten Schaltelement parallel zu einem der Längsstäbe einer Antenne mit Vogelkäfigstruktur angeordnet werden. Bei dieser Anordnung kann eine Induktivität des Längsstabs in Abhängigkeit von dem Schaltzustand des Schaltelements erhöht werden. Eine derartige Erhöhung der Induktivität kann die Symmetrie oder Homogenität der Feldverteilung verbessern.

Bei einem weiteren Ausführungsbeispiel sind die mehreren Schaltelemente unabhängig voneinander schaltbar. Dadurch kann eine genaue Einstellung der Feldverteilung und somit eine Kompensation von Positionsungenauigkeit oder sonstigen Asymmetrien oder Inhomogenitäten der Feldverteilung ermöglicht werden.

Gemäß der vorliegenden Erfindung wird ferner eine Magnetresonanzanlage mit einer Antennenanordnung gemäß Anspruch 8 bereitgestellt. Eine Symmetrie einer Feldverteilung der Antennenanordnung ist erfindungsgemäß durch eine Positionsungenauigkeit der Antennenanordnung in der Magnetresonanzanlage beeinträchtigt. Die Antennenanordnung umfasst mehrere Antennenschwingkreiselemente, mehrere Schaltelemente und eine Steuervorrichtung, welche mit den mehreren Schaltelementen zur Ansteuerung der Schaltelemente gekoppelt ist. Jedem Antennenschwingkreiselement der mehreren Antennenschwingkreiselemente ist jeweils ein Schaltelement der mehreren Schaltelemente zugeordnet. Ein jeweiliges Schaltelement ist ausgestaltet, dass zugeordnete Antennenschwingkreiselement in Abhängigkeit von einem Schaltzustand des Schaltelements mit der Antennenanordnung funktionsfähig zu koppeln. Die Steuervorrichtung ist ausgestaltet, eine Symmetrieinformation der Feldverteilung in der Antennenanordnung zu messen und automatisch die mehreren Schaltelemente anhand der gemessenen Symmetrieinformation derart einzustellen, dass eine Symmetrie der Feldverteilung im Inneren der Antennenanordnung erhöht wird.

Die Antennenanordnung ist ausgestaltet, das zuvor beschriebene Verfahren auszuführen und umfasst daher auch die im Zusammenhang mit dem Verfahren zuvor beschriebenen Vorteile.

Die Schaltelemente können beispielsweise Dioden umfassen, deren Schaltzustand mittels einer Vorspannung einstellbar ist. Die Vorspannung kann von der Steuervorrichtung eingestellt werden. Alternativ oder zusätzlich können die Schaltelemente Transistoren umfassen, welche von der Steuervorrichtung angesteuert werden.

Die Antennenanordnung kann zum Beispiel eine Vogelkäfigstruktur aufweisen. Bei einer beispielhaften Antennenanordnung umfasst die Antennenanordnung zwei Endringe. Jeder der Endringe weist in Umfangsrichtung mehrere voneinander isolierte leitfähige Flächen auf. Die mehreren leitfähigen Flächen sind somit voneinander galvanisch getrennt. Ein Antennenschwingkreiselement der mehreren Antennenschwingkreiselementen kann beispielsweise einen Kondensator umfassen. Der Kondensator ist zwischen zwei leitfähigen Flächen von einem der Endringe angeordnet. Beispielsweise kann der Kondensator zusammen mit seinem zugeordneten Schaltelement derart zwischen den zwei leitfähigen Flächen angeordnet sein, dass eine kapazitive Kopplung zwischen den beiden leitfähigen Flächen bei geschlossenem Schaltelement um die Kapazität des Kondensators erhöht wird. Dadurch kann die kapazitive Kopplung zwischen den leitfähigen Flächen der Endringe verändert werden.

Die zwei leitfähigen Flächen von dem einen der Endringe können eine kapazitive Kopplung zueinander aufweisen, beispielsweise aufgrund ihrer geometrischen Anordnung zueinander oder aufgrund von dazwischen angeordneten kapazitiven Elementen. Der schaltbare Kondensator kann eine Kapazität im Bereich von 1% bis 20% der kapazitiven Kopplung der zwei leitfähigen Flächen aufweisen. Insbesondere kann der schaltbare Kondensator eine Kapazität im Bereich von 5% bis 10% der kapazitiven Kopplung der zwei leitfähigen Flächen aufweisen. Eine Änderung der kapazitiven Kopplung zwischen den zwei leitfähigen Flächen im Bereich von beispielsweise 1% bis 20% ist im Allgemeinen ausreichend, um Positionierungstoleranzen der Antennenanordnung in Bezug zum Hochfrequenzschirm in einer Magnetresonanzanlage auszugleichen.

Bei einer weiteren beispielhaften Antennenanordnung umfasst die Antennenanordnung mehrere Längsstäbe. Ein Antennenschwingkreiselement der mehreren Antennenschwingkreiselemente kann beispielsweise ein induktives Element umfassen. Das induktive Element wird parallel zu einem der mehreren Längsstäbe in Längsrichtung angeordnet. Beispielsweise kann das induktive Element zusammen mit seinem zugeordneten Schaltelement derart parallel zu einem der mehreren Längsstäbe angeordnet sein, dass eine Induktivität des Längsstabs erhöht wird.

Das induktive Element kann beispielsweise eine Induktivität im Bereich von 1% bis 20% der Induktivität des Längsstabs aufweisen. Insbesondere kann das induktive Element eine Induktivität im Bereich von 5% bis 10% der Induktivität des Längsstabs aufweisen. Dadurch kann die Induktivität des Längsstabs in Abhängigkeit von einem Schaltzustand des Schaltelements um die Induktivität des induktiven Elements erhöht werden. Eine derartige Änderung der Induktivität eines Längsstabs im Bereich von beispielsweise 1% bis 20% ist im Allgemeinen ausreichend, um Positionierungstoleranzen der Antennenanordnung in Bezug zum Hochfrequenzschirm in einer Magnetresonanzanlage auszugleichen.

Die Magnetresonanzanlage kann ferner beispielsweise eine Hochfrequenzsteuervorrichtung zur Ansteuerung der Antennenanordnung, eine Gradientensteuereinheit, eine Bildsequenzsteuerung und eine Recheneinheit umfassen, die ausgebildet sind, um MR-Daten eines vorbestimmten Volumenabschnitts innerhalb eines Untersuchungsobjekts zu erfassen. Erfindungsgemäß ist die Symmetrie der Feldverteilung durch eine Positionsungenauigkeit der Antennenanordnung in der Magnetresonanzanlage beeinträchtigt. Die Positionsungenauigkeit der Antennenanordnung umfasst eine Positionsungenauigkeit einer Anordnung der Antennenanordnung in Bezug auf einen Hochfrequenzschirm, wobei das Einstellen der mehreren Schaltelemente derart, dass eine Symmetrie der Feldverteilung im Innern der Antennenanordnung erhöht wird, die Positionsungenauigkeit zumindest teilweise kompensiert.

Die Magnetresonanzanlage ist ausgestaltet, das zuvor beschriebene Verfahren auszuführen und umfasst daher auch die im Zusammenhang mit dem Verfahren zuvor beschriebenen Vorteile.

Des Weiteren betrifft die vorliegende Erfindung ein Computerprogrammprodukt gemäß Anspruch 14.

Mit diesem Computerprogrammprodukt können alle oder verschiedene vorab beschriebene Ausführungsformen des erfindungsgemäßen Verfahrens ausgeführt werden, wenn das Computerprogrammprodukt in der Steuervorrichtung läuft. Dabei benötigt das Computerprogrammprodukt eventuell Programmmittel, z.B. Bibliotheken und Hilfsfunktionen, um die entsprechenden Ausführungsformen des Verfahrens zu realisieren. Mit anderen Worten soll mit dem auf das Computerprogrammprodukt gerichteten Anspruch insbesondere eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (z.B. C++), der noch compiliert und gebunden oder der nur interpretiert werden muss, oder um einen ausführbaren Softwarecode handeln, der zur Ausführung nur noch in die entsprechende Recheneinheit zu laden ist.

Schließlich betrifft die vorliegende Erfindung einen elektronisch lesbaren Datenträger gemäß Anspruch 15, z.B. eine DVD, ein Magnetband, eine Festplatte oder einen USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuervorrichtung bzw. Recheneinheit einer Magnetresonanzanlage gespeichert werden, können alle erfindungsgemäßen Ausführungsformen des vorab beschriebenen Verfahrens durchgeführt werden.

Nachfolgend wird die vorliegende Erfindung anhand erfindungsgemäßer Ausführungsformen unter Bezugnahme auf die Figuren im Detail beschrieben. Gleiche Bezugszeichen in den unterschiedlichen Figuren bezeichnen gleiche oder ähnliche Komponenten.
FIG 1 stellt schematisch eine Magnetresonanzanlage gemäß einer Ausführungsform der Erfindung dar.
FIG 2 zeigt schematisch eine Antennenanordnung gemäß einer Ausführungsform der vorliegenden Erfindung.
FIG 3 zeigt schematisch einen Ausschnitt einer Antennenanordnung gemäß einer Ausführungsform der vorliegenden Erfindung.
FIG 4 zeigt schematisch einen Ausschnitt einer Antennenanordnung gemäß einer weiteren Ausführungsform der vorliegenden Erfindung.
FIG 5 zeigt schematisch einen Ausschnitt einer Antennenanordnung gemäß einer weiteren Ausführungsform der vorliegenden Erfindung.
FIG 6 zeigt schematisch ein Schaltelement mit einer Ansteuerung gemäß einer Ausführungsform der vorliegenden Erfindung.
FIG 7 zeigt schematisch ein Ablaufdiagramm mit Verfahrensschritten eines Verfahrens gemäß einer Ausführungsform der vorliegenden Erfindung.

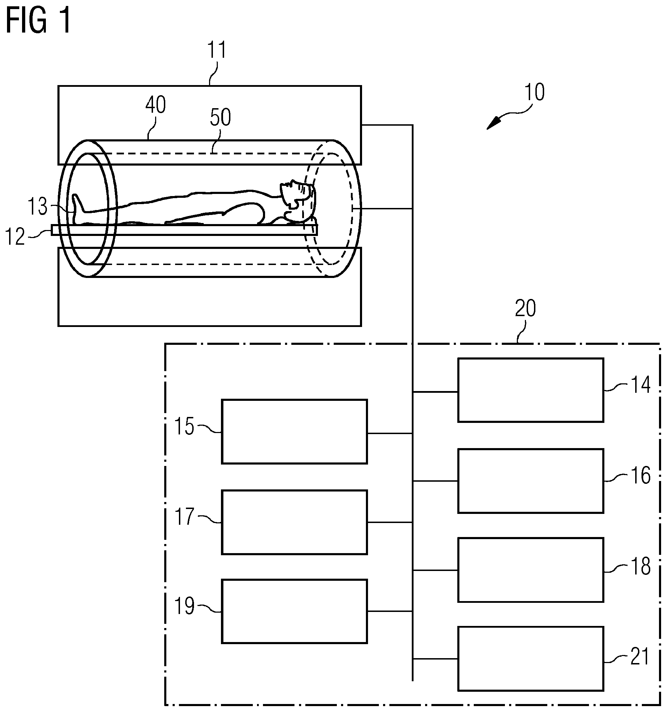

FIG 1 stellt schematisch eine Magnetresonanzanlage 10 (MR-Anlage) dar. Die Magnetresonanzanlage 10 weist einen Magneten 11 zur Erzeugung eines Polarisationsfelds B0 auf. Eine auf einer Liege 12 angeordnete Untersuchungsperson 13 kann in eine zylinderförmiger Öffnung in dem Magneten 11 gefahren werden, um dort ortskodierte Magnetresonanzsignale bzw. MR-Daten von der Untersuchungsperson 13 aufzunehmen. Rund um die zylinderförmiger Öffnung ist ferner eine zylinderförmige Antennenanordnung 50 zur Erzeugung von Hochfrequenzsignalen, insbesondere Hochfrequenzpulsen (HF-Pulse), vorgesehen. Um eine definierte Umgebung für eine Felderzeugung durch die Antennenanordnung 50 bereitzustellen, ist ferner ein zylinderförmiger Hochfrequenzschirm 40 rund um die Antennenanordnung 50 vorgesehen. Durch Einstrahlen von Hochfrequenzpulsen mit der Antennenanordnung 50 und Schalten von Magnetfeldgradienten kann die durch das Polarisationsfeld B0 erzeugte Magnetisierung aus der Gleichgewichtslage ausgelenkt und ortskodiert werden, und die sich ergebende Magnetisierung wird von Empfangsspulen detektiert. Wie durch Einstrahlen der HF-Pulse und durch Schalten von Magnetfeldgradienten in verschiedenen Kombinationen und Reihenfolgen Magnetresonanzbilder (MR-Bilder) erzeugt werden können, ist dem Fachmann grundsätzlich bekannt und wird hier nicht näher erläutert.

Die Magnetresonanzanlage 10 weist weiterhin eine Steuerung 20 auf, die zur Steuerung der Magnetresonanzanlage 10 verwendet werden kann. Die Steuerung 20 weist eine Gradientensteuereinheit 15 zur Steuerung und Schaltung der notwendigen Magnetfeldgradienten auf. Eine Hochfrequenzsteuervorrichtung (HF-Steuervorrichtung) 14 ist für die Steuerung der Antennenanordnung 50 und die Generierung der HF-Pulse zur Auslenkung der Magnetisierung vorgesehen. Eine Bildsequenzsteuerung 16 steuert die Abfolge der Magnetfeldgradienten und HF-Pulse und damit indirekt die Gradientensteuereinheit 15 und die HF-Steuervorrichtung 14. Über eine Eingabeeinheit 17 kann eine Bedienperson die Magnetresonanzanlage 10 steuern und auf einer Anzeigeeinheit 18 können MR-Bilder und sonstige zur Steuerung notwendige Informationen angezeigt werden. Eine Recheneinheit 19 mit mindestens einer Prozessoreinheit (nicht gezeigt) ist vorgesehen zur Steuerung der verschiedenen Einheiten in der Steuerung 20 und zur Durchführung von Rechenoperationen. Weiterhin ist eine Speichereinheit 21 vorgesehen, in der beispielsweise Programmmodule bzw. Programme abgespeichert sein können, die, wenn sie von der Recheneinheit 19 bzw. ihrer Prozessoreinheit ausgeführt werden, den Ablauf der Magnetresonanzanlage 10 steuern können. Die Recheneinheit 19 ist ausgebildet, um aus den erfassten Magnetresonanzsignalen die MR-Bilder zu berechnen.

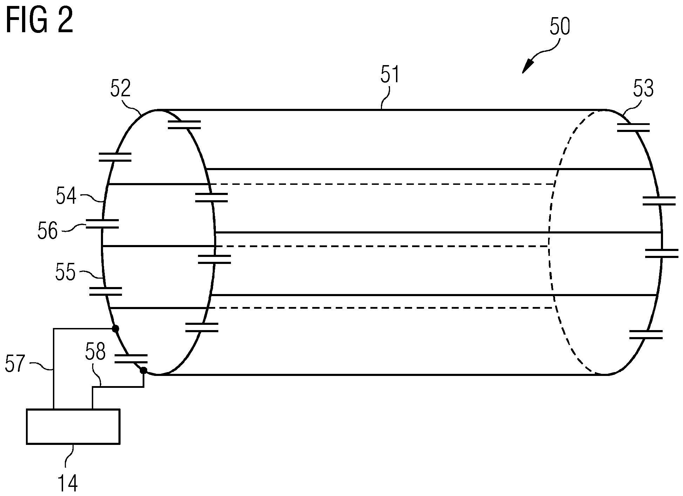

FIG 2 zeigt in Form eines dreidimensionalen Drahtmodells einen beispielhaften Aufbau der Antennenanordnung 50. Die Antennenanordnung 50 der FIG 2 weist eine sogenannte Vogelkäfigstruktur (Birdcage structure) auf. Eine solche Vogelkäfigstruktur besteht aus einer Anzahl von auf einer zylinderförmigen Oberfläche angeordneten, äquidistanten, parallel verlaufenden Antennen-Längsstäben 51. In dem Beispiel der FIG 2 weist die Antennenanordnung 50 acht Längsstäbe 51 auf. Die Antennenanordnung 50 kann jedoch eine beliebige Anzahl von Antennen-Längsstäben 51 aufweisen, beispielsweise sechs oder mehr als acht, beispielsweise zwölf oder 16. Diese Längsstäbe 51 sind endseitig jeweils durch Antennen-Endringe 52,53 hochfrequenzmäßig untereinander verbunden. "Hochfrequenzmäßig verbunden" bedeutet in diesem Zusammenhang, dass nicht zwingend eine galvanische Verbindung, sondern lediglich eine für Hochfrequenzströme durchlässige Verbindung zwischen den Längsstäben besteht. Beispielsweise befinden sich in den Antennen-Endringen 52,53 jeweils zwischen zwei Anschlussstellen 54,55 benachbarter Antennen-Längsstäbe 51 Resonanzkondensatoren 56. Die Anschlussstellen 54,55 können beispielsweise durch leitfähige Flächen des jeweiligen Endrings 52,53 gebildet werden. Die Resonanzkondensatoren 56 können beispielsweise durch gegenüberliegende Flächen dieser leitfähigen Flächen 54,55 gebildet werden. Alternativ oder zusätzlich können Resonanzkondensatoren 56 als diskrete Bauelemente zwischen den leitfähigen Flächen angeordnet sein. Die Längsstäbe 51 besitzen aufgrund ihrer physikalischen Ausdehnung in Längsrichtung jeweils eine entsprechende Induktivität.

In dem in FIG 2 dargestellten Beispiel sind die Endringe 52,53 jeweils rund. Alternativ können die Endringe 52,53 auch aus jeweils zwischen zwei Antennen-Längsstäben 51 verlaufenden geraden Abschnitten bestehen.

Die Antennenanordnung 50 ist über Anschlussleitungen 57,58 mit der Hochfrequenzsteuervorrichtung 14 verbunden. Die Anschlussleitungen 57,58 sind jeweils an benachbarten Anschlussstellen neben einem Resonanzkondensator 56 angeschlossen. Über diese Anschlussleitungen 57,58 erfolgt nicht nur die Einspeisung der Hochfrequenzpulse im Sendebetrieb, sondern auch ein Abgriff der aufgefangenen Magnetresonanzsignale im Empfangsbetrieb.

Für eine optimale Leistungsfähigkeit der Antennenanordnung 50 ist eine genaue Anordnung bzw. ein genaues Zusammenspiel von der Antennenanordnung 50 und dem Hochfrequenzschirm 40 entscheidend. Unter anderem ist eine genaue Anordnung der Antennenanordnung 50 in dem Hochfrequenzschirm 40, beispielsweise ein gleichmäßiger Abstand zwischen der Antennenanordnung 50 und dem Hochfrequenzschirm 40, entscheidend für eine Homogenität oder Symmetrie eines von der Antennenanordnung 50 erzeugten Hochfrequenzfeldes. Ferner können eine Resonanzfrequenz oder mehrere Resonanzfrequenzen und deren Entkopplung der Antennenanordnung 50 durch die Anordnung der Antennenanordnung 50 in dem Hochfrequenzschirm 40 beeinflusst werden. Die Leistungsfähigkeit der Antennenanordnung 50 kann somit durch mechanische Toleranzen beim Einbau der Antennenanordnung 50 beeinträchtigt werden. Darüber hinaus kann die Leistungsfähigkeit der Antennenanordnung 50 im Betrieb der Magnetresonanzanlage 10 durch beispielsweise das Anordnen des Patienten 13 innerhalb der Antennenanordnung 50 beeinträchtigt werden, da eine Feldverteilung des von der Antennenanordnung 50 erzeugten Feldes von dem Patienten 13 beeinflusst werden kann.

Insbesondere um die mechanischen Toleranzen zu kompensieren, weist die Antennenanordnung 50 schaltbare Antennenschwingkreiselemente auf.

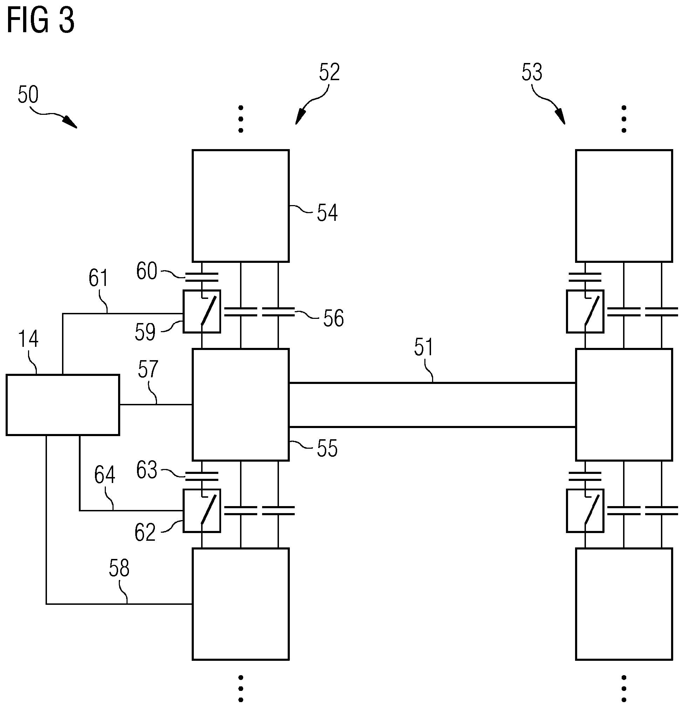

FIG 3 zeigt einen Ausschnitt der Antennenanordnung 50 der FIG 2. FIG 3 zeigt nur einige der Anschlussstellen 54,55 der Endringe 52,53 und lediglich einen Antennen-Längsstab 51 der mehreren Antennen-Längsstäbe. In dem Beispiel der FIG 3 sind zwischen jeweils zwei benachbarten Anschlussstellen 54,55 jeweils zwei Resonanzkondensatoren 56 gezeigt. Die Anzahl dieser Resonanzkondensatoren 56 zwischen jeweils zwei benachbarten Anschlussstellen 54,55 ist beliebig. Wie zuvor beschrieben, kann die Kapazität dieser Resonanzkondensatoren 56 auch beispielsweise durch die Anschlussstellen 54,55 selbst erzeugt werden. Die Anschlussstellen 54,55 können, wie in FIG 3 gezeigt, leitfähige Flächen umfassen, welche sich an einer Seite gegenüber liegen und somit eine Kapazität ausbilden können. Die leitfähigen Flächen 54,55 sind mit dem Längsstab 51 leitend verbunden, wobei der Längsstab 51 wiederum als eine leitfähige Fläche ausgebildet sein kann.

Als schaltbare Antennenschwingkreiselemente sind in der FIG 3 Kombinationen aus einem Schaltelement und einem Kondensator gezeigt. Beispielsweise ist zwischen der leitfähigen Fläche 54 und der leitfähigen Fläche 55 ein Schaltelement 59 in Reihe mit einem Kondensator 60 als schaltbares Antennenschwingkreiselement vorgesehen. Auf der anderen Seite der leitfähigen Fläche 55 ist zu der dort benachbarten leitfähigen Fläche eine Reihenschaltung eines Schaltelements 62 und eines Kondensators 63 als schaltbares Antennenschwingkreiselement vorgesehen. An dem Endring 53 sind ebenfalls entsprechende schaltbare Antennenschwingkreiselemente zwischen den leitfähigen Flächen vorgesehen. Im geschlossenen Zustand des Schaltelements 59 wirkt sich die Kapazität des Kondensators 60 zusätzlich auf die kapazitive Kopplung zwischen der leitfähigen Fläche 54 und der leitfähigen Fläche 55 aus. Die Schaltelemente 59,62 sind von der Steuervorrichtung 14 steuerbar. Dazu sind zwischen der Steuervorrichtung 14 und dem Schaltelement 59 eine Steuerleitung 61 und zwischen der Steuervorrichtung 14 und dem Schaltelement 62 eine Steuerleitung 64 vorgesehen.

Mithilfe der schaltbaren Antennenschwingkreiselemente kann die Feldverteilung im Inneren der Antennenanordnung 50 eingestellt werden, um beispielsweise eine Symmetrie oder Homogenität der Feldverteilung zu erhöhen und somit mechanische Toleranzen der Positionierung der Antennenanordnung 50 in dem Hochfrequenzschirm 40 kompensieren. Bereits mit nur wenigen schaltbaren Antennenschwingkreiselementen kann eine zufriedenstellende Einstellung der Feldverteilung im Inneren der Antennenanordnung 50 erreicht werden. Beispielsweise können in jedem der Endringe 52,53 lediglich zwei bis vier schaltbare Antennenschwingkreiselemente vorgesehen sein. Natürlich kann in jedem der Endringe 52,53 zwischen jeder der Anschlussstellen 54,55 ein jeweiliges schaltbares Antennenschwingkreiselement vorgesehen sein.

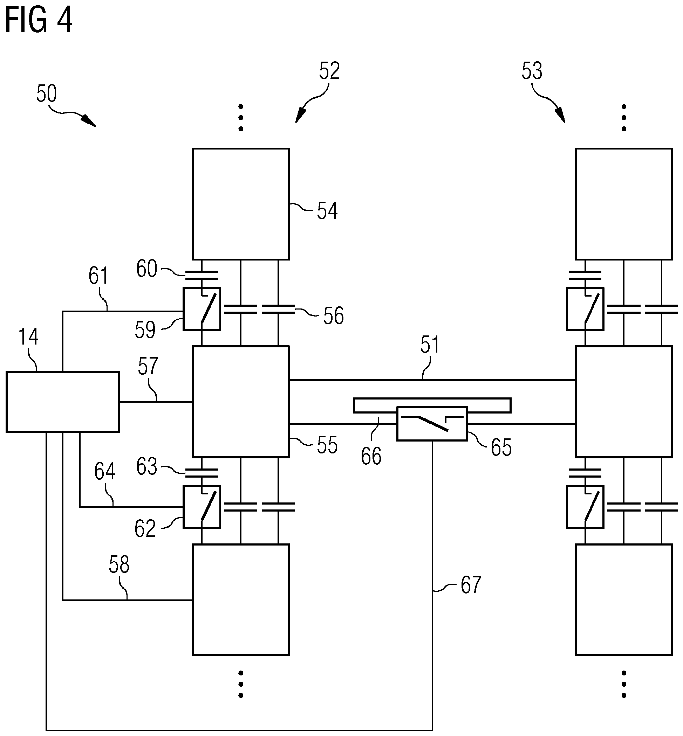

FIG 4 zeigt ein weiteres Beispiel eines schaltbaren Antennenschwingkreiselements. Das schaltbare Antennenschwingkreiselement in FIG 4 umfasst eine Kombination aus einem Schaltelement 65 und einem induktiven Element 66. Das induktive Element 66 kann beispielsweise eine Leiterbahn oder ein flächiger Leiter parallel zu dem Längsstab 51 sein. Im geschlossenen Zustand des Schaltelements 65 wirkt sich die Induktivität des induktiven Elements 66 zusätzlich auf die Induktivität des Antennen-Längsstab 51 aus. Das Schaltelement 65 ist von der Steuervorrichtung 14 steuerbar. Dazu ist zwischen der Steuervorrichtung 14 und dem Schaltelement 65 eine Steuerleitung 67 vorgesehen. In einem Antennen-Längsstab 51 können auch mehrere dieser schaltbaren Antennenschwingkreiselemente vorgesehen sein.

Mit dem schaltbaren Antennenschwingkreiselemente in dem Antennen-Längsstab 51 kann die Feldverteilung im Inneren der Antennenanordnung 50 eingestellt werden, um beispielsweise eine Symmetrie oder Homogenität der Feldverteilung zu erhöhen und somit mechanische Toleranzen der Positionierung der Antennenanordnung 50 in dem Hochfrequenzschirm 40 zu kompensieren. Bereits mit nur wenigen schaltbaren Antennenschwingkreiselementen kann eine zufriedenstellende Einstellung der Feldverteilung im Inneren der Antennenanordnung 50 erreicht werden. Beispielsweise kann in nur wenigen der Antennen-Längsstäbe 51 ein jeweiliges schaltbares Antennenschwingkreiselement vorgesehen sein, beispielsweise nur in zwei oder vier der Antennen-Längsstäbe 51. Selbstverständlich kann in jedem der Längsstäbe 51 ein entsprechendes schaltbares Antennenschwingkreiselement vorgesehen werden.

Die Antennenschwingkreiselemente zwischen den leitfähigen Flächen 54,55 und in den Antennen-Längsstäben 51 sind unabhängig voneinander von der Steuervorrichtung 14 ansteuerbar.

Mit den zuvor beschriebenen schaltbaren Antennenschwingkreiselementen in der Antennenanordnung 50 können beispielsweise mechanische Toleranzen, die sich aus dem Zusammenspiel zwischen dem Hochfrequenzschirm 40 und der Antennenanordnung 50 ergeben, kompensiert werden. Diese schaltbaren Antennenschwingkreiselemente können verwendet werden, um Resonanzfrequenzen und deren Entkopplung einzustellen. Die Resonanzfrequenzen und deren Entkopplung können beispielsweise mit der Hochfrequenzsteuervorrichtung 14 beim Ansteuern der Antennenanordnung 50 gemessen werden und die schaltbaren Antennenschwingkreiselemente entsprechend angesteuert werden, um geeignete Zielwerte für die Resonanzfrequenzen und deren Entkopplung zu erreichen. Dabei können die schaltbaren Antennenschwingkreiselemente beispielsweise mithilfe eines Optimierungsverfahrens, beispielsweise mithilfe eines Gradientenabstiegsverfahrens, angesteuert werden, um ein gewünschte Resonanzfrequenzen und deren Entkopplung einzustellen. Alternativ können auch alle möglichen Schaltkombinationen der schaltbaren Antennenschwingkreiselemente ausprobiert werden und die geeignetste Schaltkombination für den Betrieb ausgewählt werden.

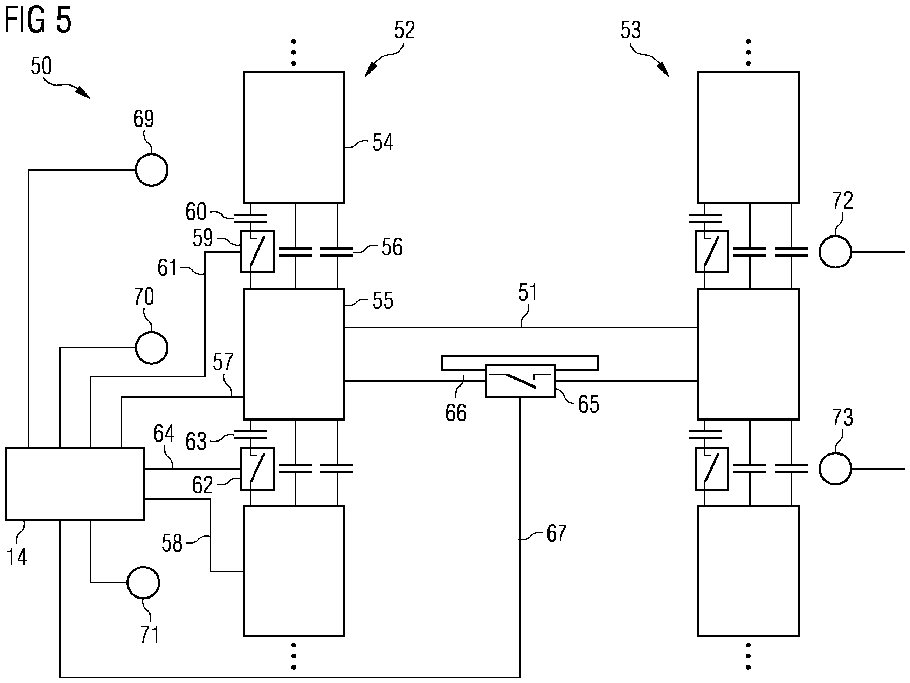

Neben den Resonanzfrequenzen und der Entkopplung kann beispielsweise auch eine Stromverteilung in der Antennenanordnung 50 als ein Optimierungskriterium verwendet werden. Dazu kann jedem Schaltelement ein Stromsensor zugeordnet werden. FIG 5 zeigt eine derartige Anordnung, bei welcher den Schaltelementen in dem Endring 53 jeweils ein entsprechender Stromsensor 72,73 zugeordnet ist. Alternativ oder zusätzlich können auch Stromsensoren vorgesehen werden, welche einen Strom in den Anschlussstellen 54,55 messen. In FIG 5 sind beispielsweise Stromsensoren 69-71 zum Messen von jeweiligen Strömen in den Anschlussstellen 54,55 in dem Endring 53 gezeigt. Die Stromsensoren 69-73 können mit der Hochfrequenzsteuervorrichtung 14 gekoppelt sein. Die Stromsensoren 69-73 können jeweils eine sogenannte Pick-up-Spule umfassen. Unter Berücksichtigung der Informationen von den Stromsensoren 69-73 können die schaltbaren Antennenschwingkreiselemente so eingestellt werden, dass eine homogene Stromverteilung und damit auch eine homogene Feldverteilung erreicht wird. Auswirkungen von Asymmetrien beim Einbau können damit zumindest teilweise kompensiert werden. Wenn beispielsweise die Antennenanordnung 50 nicht mittig im Hochfrequenzschirm 40 platziert ist, kann dies zu einer Asymmetrie im B1-Feld in transversale Richtung führen. Durch geeignete Ansteuerung der schaltbaren Antennenschwingkreiselemente kann diese Asymmetrie kompensiert werden. Es ist klar, dass nicht im Bereich von jedem schaltbaren Antennenschwingkreiselement und jeder Anschlussstelle ein entsprechender Stromsensor vorgesehen sein muss, um eine homogenere Stromverteilung zu erreichen. Bereits mit einer geringeren Anzahl von Stromsensoren können Asymmetrien und Inhomogenitäten zumindest teilweise erfasst und somit kompensiert werden.

Auch im Betrieb der Magnetresonanzanlage 10, d.h., wenn beispielsweise ein Patient 13 in die Magnetresonanzanlage 10 eingefahren wird, können die schaltbaren Antennenschwingkreiselemente von der Hochfrequenzsteuervorrichtung 14 angesteuert werden, um beispielsweise Rückwirkungen des Patienten 13 auf die Stromverteilung in der Antennenanordnung 50 zu kompensieren. Dazu können beispielsweise wiederum die zuvor beschriebenen Stromsensoren verwendet werden. Alternativ oder zusätzlich können neben den Stromsensoren auch Sensoren zur Messung des B1-Feldes an verschiedenen Stellen zur Bewertung des B1-Feldes und Einstellung der schaltbaren Antennenschwingkreiselemente herangezogen werden. Beispielsweise kann das B1-Feld mit fest eingebauten Sensoren oder mit ortsveränderlichen Sensoren an bestimmten Stellen gemessen werden und eine sogenannte B1-Map erzeugt werden. Auf der Grundlage dieser B1-Map kann die B1-Feldverteilung von der Hochfrequenzsteuervorrichtung 14 beurteilt werden und die schaltbaren Antennenschwingkreiselemente zur Verbesserung des B1-Feldes entsprechend angesteuert werden.

Zusammenfassend können mithilfe der schaltbaren Antennenschwingkreiselemente mechanische Toleranzen und deren Einfluss auf die Abstimmung der Antennenanordnung 50 automatisiert und kostengünstig kompensiert werden.

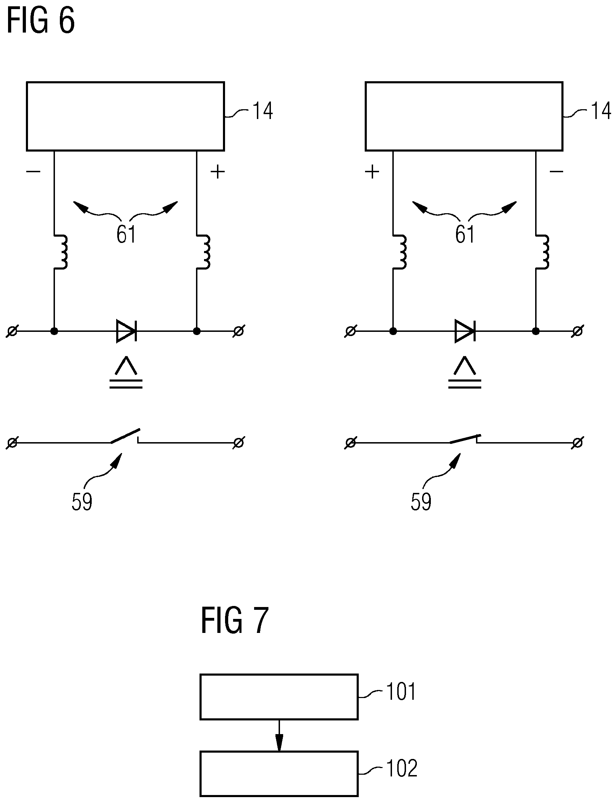

FIG 6 zeigt schematisch ein Beispiel für ein Schaltelement, beispielsweise für das Schaltelement 59. In dem in FIG 6 gezeigten Beispiel umfasst das Schaltelement eine Diode. Die Diode kann insbesondere eine sogenannte PIN-Diode umfassen. Alternativ oder zusätzlich sind andere schaltbare Halbleiterelemente als Schaltelemente verwendbar, beispielsweise Transistoren.

Die Schaltelemente 59,62,65 in den Endringen 52,53 und den Antennen-Längsstäben 51 sind getrennt voneinander einzelnen über entsprechende Steuerleitungen ein- und ausschaltbar. In FIG 6 links wird von der Steuervorrichtung 14 über die Steuerleitungen 61 an die Anode der Diode eine gegenüber der Kathode negative Spannung angelegt. Dadurch sperrt die Diode und das Schaltelement 59 ist für Hochfrequenzströme nicht durchlässig, das Schaltelement 59 ist somit "offen". In FIG 6 rechts wird von der Steuervorrichtung 14 über die Steuerleitungen 61 an die Anode der Diode eine gegenüber der Kathode positive Spannung angelegt. Dadurch leitet die Diode und das Schaltelement 59 ist für Hochfrequenzströme durchlässig, das Schaltelement 59 ist somit "geschlossen".

In FIG 7 ist ein Ablaufdiagramm eines Verfahrens mit Schritten 101 und 102 dargestellt. Das Verfahren kann beispielsweise mittels der Hochfrequenzsteuervorrichtung 14 durchgeführt werden.

Im Schritt 101 wird eine Symmetrieinformation der Feldverteilung in der Antennenanordnung 50 gemessen. Die Symmetrieinformation kann beispielsweise ein Maß für eine Homogenität oder Symmetrie eines von der Antennenanordnung 50 erzeugten B1-Feldes anzeigen. Zur Bestimmung der Symmetrieinformation kann beispielsweise eine Stromverteilung in der Antennenanordnung 50 gemessen werden, Resonanzfrequenzen und deren Entkopplung beim Einspeisen von Hochfrequenzsignalen in die Antennenanordnung 50 gemessen werden oder das von der Antennenanordnung 50 erzeugten B1-Feld an verschiedenen Positionen gemessen werden. Im Schritt 102 werden die mehreren Schaltelemente 59,62,65 in den Endringen 52,53 und in den Antennen-Längsstäben 51 anhand der gemessenen Symmetrieinformation derart automatisch eingestellt, dass eine Symmetrie der Feldverteilung im Inneren der Antennenanordnung 50 erhöht wird. Die Verfahrensschritte 101 und 102 können mehrfach interaktiv durchgeführt werden, um eine Optimierung der Symmetrie der Feldverteilung im Inneren der Antennenanordnung 50 zu erreichen.

## Patentansprüche

1. Verfahren zum Einstellen einer Feldverteilung einer Antennenanordnung einer Magnetresonanzanlage,
wobei die Antennenanordnung (50) eine zylinderförmige Antennenanordnung (50) zur Erzeugung von Hochfrequenzsignalen ist, die einen zylinderförmigen Hochfrequenzschirm (40) rund um die Antennenanordnung (50) aufweist,
wobei die Antennenanordnung (10) mehrere Antennenschwingkreiselemente (60, 63, 66) und mehrere Schaltelemente (59, 62, 65) umfasst, wobei jedem Antennenschwingkreiselement der mehreren Antennenschwingkreiselemente (60, 63, 66) jeweils ein Schaltelement der mehreren Schaltelemente (59, 62, 65) zugeordnet ist, wobei ein jeweiliges Schaltelement ausgestaltet ist, das zugeordnete Antennenschwingkreiselement in Abhängigkeit von einem Schaltzustand des Schaltelements mit der Antennenanordnung (50) funktionsfähig zu koppeln, wobei das Verfahren umfasst:
- Messen (101) einer Symmetrieinformation der Feldverteilung in der Antennenanordnung (50), und
- automatisches Einstellen (101) der mehreren Schaltelemente (59, 62, 65) anhand der gemessenen Symmetrieinformation derart, dass eine Symmetrie der Feldverteilung im Innern der Antennenanordnung (50) erhöht wird, **dadurch gekennzeichnet, dass** die Symmetrie der Feldverteilung durch eine Positionsungenauigkeit der Antennenanordnung (50) in der Magnetresonanzanlage (10) beeinträchtigt wird, wobei die Positionsungenauigkeit der Antennenanordnung (50) eine Positionsungenauigkeit einer Anordnung der Antennenanordnung (50) in Bezug auf den Hochfrequenzschirm (40) umfasst, wobei das Einstellen der mehreren Schaltelemente (59, 62, 65) derart, dass eine Symmetrie der Feldverteilung im Innern der Antennenanordnung (50) erhöht wird, die Positionsungenauigkeit zumindest teilweise kompensiert.

2. Verfahren nach Anspruch 1, wobei das Messen der Symmetrieinformation ein Messen einer Resonanzfrequenz der Antennenanordnung (50) umfasst, wobei die mehreren Schaltelemente (59, 62, 65) anhand der Resonanzfrequenz derart eingestellt werden, dass eine Symmetrie der Feldverteilung im Innern der Antennenanordnung (50) erhöht wird.

3. Verfahren nach einem der vorhergehenden Ansprüche, wobei das Messen der Symmetrieinformation ein Messen eines B1-Feldes in der Antennenanordnung (50) umfasst, wobei die mehreren Schaltelemente (59, 62, 65) anhand der B1-Feld-Messung derart eingestellt werden, dass eine Symmetrie der Feldverteilung im Innern der Antennenanordnung (50) erhöht wird.

4. Verfahren nach einem der vorhergehenden Ansprüche, wobei das Messen der Symmetrieinformation ein Messen einer Stromverteilung in der Antennenanordnung (50) umfasst, wobei die mehreren Schaltelemente (59, 62, 65) anhand der Stromverteilung derart eingestellt werden, dass eine Symmetrie der Feldverteilung im Innern der Antennenanordnung (50) erhöht wird.

5. Verfahren nach einem der vorhergehenden Ansprüche, wobei ein Antennenschwingkreiselement der mehreren Antennenschwingkreiselemente (60, 63, 66) einen Kondensator (60, 63) umfasst, wobei das Verfahren ferner umfasst:
- Koppeln des Kondensators (60, 63) mittels des zugeordneten Schaltelements mit der Antennenanordnung (50), um eine Kapazität der Antennenanordnung (50) zur Erhöhung der Symmetrie der Feldverteilung in der Antennenanordnung (50) zu verändern.

6. Verfahren nach einem der vorhergehenden Ansprüche, wobei ein Antennenschwingkreiselement der mehreren Antennenschwingkreiselemente (60, 63, 66) ein induktives Element (66) umfasst, wobei das Verfahren ferner umfasst:
- Koppeln des induktiven Elements (66) mittels des zugeordneten Schaltelements mit der Antennenanordnung (50), um eine Induktivität der Antennenanordnung (50) zur Erhöhung der Symmetrie der Feldverteilung in der Antennenanordnung (50) zu verändern.

7. Verfahren nach einem der vorhergehenden Ansprüche, wobei die mehreren Schaltelemente (59, 62, 65) unabhängig voneinander schaltbar sind.

8. Magnetresonanzanlage mit einer Antennenanordnung (50), wobei die Antennenanordnung (50) eine zylinderförmige Antennenanordnung (50) zur Erzeugung von Hochfrequenzsignalen ist, die einen zylinderförmigen Hochfrequenzschirm (40) rund um die Antennenanordnung (50) aufweist, wobei die Antennenanordnung (50) umfasst:
- mehrere Antennenschwingkreiselemente (60, 63, 66),
- mehrere Schaltelemente (59, 62, 65), wobei jedem Antennenschwingkreiselement der mehreren Antennenschwingkreiselemente (60, 63, 66) jeweils ein Schaltelement der mehreren Schaltelemente (59, 62, 65) zugeordnet ist, wobei ein jeweiliges Schaltelement ausgestaltet ist, das zugeordnete Antennenschwingkreiselement in Abhängigkeit von einem Schaltzustand des Schaltelements mit der Antennenanordnung (50) funktionsfähig zu koppeln, und
- eine Steuervorrichtung (14), welche ausgestaltet ist, die folgenden Schritte auszuführen:
- Messen (101) einer Symmetrieinformation der Feldverteilung in der Antennenanordnung (50), und
- automatisches Einstellen der mehreren Schaltelemente (59, 62, 65) anhand der gemessenen Symmetrieinformation derart, dass eine Symmetrie der Feldverteilung im Innern der Antennenanordnung (50) erhöht wird, **dadurch gekennzeichnet, dass** die Symmetrie der Feldverteilung durch eine Positionsungenauigkeit der Antennenanordnung (50) in der Magnetresonanzanlage (10) beeinträchtigt wird, wobei die Positionsungenauigkeit der Antennenanordnung (50) eine Positionsungenauigkeit einer Anordnung der Antennenanordnung (50) in Bezug auf den Hochfrequenzschirm (40) umfasst, wobei das Einstellen der mehreren Schaltelemente (59, 62, 65) derart, dass eine Symmetrie der Feldverteilung im Innern der Antennenanordnung (50) erhöht wird, die Positionsungenauigkeit zumindest teilweise kompensiert.

9. Magnetresonanzanlage nach Anspruch 8, wobei die Antennenanordnung (50) zur Durchführung des Verfahrens nach einem der Ansprüche 2-7 ausgestaltet ist.

10. Magnetresonanzanlage nach Anspruch 8 oder Anspruch 9, wobei die Antennenanordnung (50) zwei Endringe (52, 53) umfasst, wobei jeder der Endringe (52, 53) in Umfangsrichtung mehrere voneinander isolierte leitfähige Flächen (54, 55) aufweist, wobei ein Antennenschwingkreiselement der mehreren Antennenschwingkreiselemente (60, 63, 66) einen Kondensator (60, 63) umfasst, wobei der Kondensator (60, 63) zwischen zwei leitfähigen Flächen (54, 55) von einem der Endringe (52, 53) angeordnet ist.

11. Magnetresonanzanlage nach Anspruch 10, wobei die zwei leitfähigen Flächen (54, 55) von dem einen der Endringe (52, 53) eine kapazitive Kopplung zueinander aufweisen, wobei der Kondensator (60, 63) eine Kapazität im Bereich von 1% bis 20%, insbesondere im Bereich von 5% bis 10%, der kapazitiven Kopplung der zwei leitfähigen Flächen (54, 55) aufweist.

12. Magnetresonanzanlage nach einem der Ansprüche 8-11, wobei die Antennenanordnung (50) mehrere Längsstäbe (51) umfasst, wobei ein Antennenschwingkreiselement der mehreren Antennenschwingkreiselemente (60, 63, 66) ein induktives Element (66) umfasst, wobei das induktive Element (66) parallel zu einem der mehreren Längsstäbe (51) in Längsrichtung angeordnet ist.

13. Magnetresonanzanlage nach Anspruch 12, wobei der eine der mehreren Längsstäbe (51) eine Induktivität aufweist, wobei das induktive Element (66) eine Induktivität im Bereich von 1% bis 20%, insbesondere im Bereich von 5% bis 10%, der Induktivität des Längsstabs (51) aufweist.

14. Computerprogrammprodukt, welches ein Programm umfasst und direkt in einen Speicher (21) einer programmierbaren Steuerung (20) einer Magnetresonanzanlage (10) nach Anspruch 8 bis 13 ladbar ist, mit Programmmitteln, um alle Schritte des Verfahrens nach einem der Ansprüche 1-7 auszuführen, wenn das Programm in der Steuerung (20) der Magnetresonanzanlage (10) ausgeführt wird.

15. Elektronisch lesbarer Datenträger mit darauf gespeicherten elektronisch lesbaren Steuerinformationen, welche derart ausgestaltet sind, dass sie bei Verwendung des Datenträgers in einer Steuerung (20) einer Magnetresonanzanlage (10) nach einem der Ansprüche 8 bis 13 das Verfahren nach einem der Ansprüche 1-7 durchführen.

## Claims

1. Method for adjusting a field distribution of an antenna arrangement of a magnetic resonance system, wherein the antenna arrangement (50) is a cylinder-shaped antenna arrangement (50) for generating radio-frequency signals, which has a cylinder-shaped radio-frequency screen (40) around the antenna arrangement (50), wherein the antenna arrangement (10) comprises multiple oscillating circuit antenna elements (60, 63, 66) and multiple switching elements (59, 62, 65), wherein each oscillating circuit antenna element out of the multiple oscillating circuit antenna elements (60, 63, 66) is in each case assigned a switching element of the multiple switching elements (59, 62, 65), wherein a respective switching element is embodied to couple the assigned oscillating circuit antenna element operatively to the antenna arrangement (50) in dependence on a switching status of the switching element, wherein the method comprises:
- measuring (101) symmetry information on the field distribution in the antenna arrangement (50), and
- automatically adjusting (101) the multiple switching elements (59, 62, 65) using the measured symmetry information such that a symmetry of the field distribution in the interior of the antenna arrangement (50) is increased, **characterised in that** the symmetry of the field distribution is negatively impacted by a positional inaccuracy of the antenna arrangement (50) in the magnetic resonance system (10), wherein
the positional inaccuracy of the antenna arrangement (50) comprises a positional inaccuracy of an arrangement of the antenna arrangement (50) relative to the radio-frequency screen (40), wherein the adjustment of the multiple switching elements (59, 62, 65) such that a symmetry of the field distribution in the interior of the antenna arrangement (50) is increased at least partially compensates the positional inaccuracy.

2. Method according to claim 1, wherein the measurement of the symmetry information comprises a measurement of a resonant frequency of the antenna arrangement (50), wherein the multiple switching elements (59, 62, 65) are adjusted using the resonant frequency such that a symmetry of the field distribution in the interior of the antenna arrangement (50) is increased.

3. Method according to one of the preceding claims, wherein the measurement of the symmetry information comprises a measurement of a B1 field in the antenna arrangement (50), wherein the multiple switching elements (59, 62, 65) are adjusted using the B1 field measurement such that a symmetry of the field distribution in the interior of the antenna arrangement (50) is increased.

4. Method according to one of the preceding claims, wherein the measurement of the symmetry information comprises a measurement of a current distribution in the antenna arrangement (50), wherein the multiple switching elements (59, 62, 65) are adjusted using the current distribution such that a symmetry of the field distribution in the interior of the antenna arrangement (50) is increased.

5. Method according to one of the preceding claims, wherein one oscillating circuit antenna element out of the multiple oscillating circuit antenna elements (60, 63, 66) comprises a capacitor (60, 63), wherein the method further comprises:
- coupling the capacitor (60, 63) to the antenna arrangement (50) by means of the assigned switching element in order to change a capacitance of the antenna arrangement (50) in order to increase the symmetry of the field distribution in the antenna arrangement (50).

6. Method according to one of the preceding claims, wherein one oscillating circuit antenna element out of the multiple oscillating circuit antenna elements (60, 63, 66) comprises an inductive element (66), wherein the method further comprises:
- coupling the inductive element (66) by means of the assigned switching element to the antenna arrangement (50) in order to change an inductance of the antenna arrangement (50) in order to increase the symmetry of the field distribution in the antenna arrangement (50).

7. Method according to one of the preceding claims, wherein the multiple switching elements (59, 62, 65) can be switched independently of one another.

8. Magnetic resonance system with an antenna arrangement (50), wherein the antenna arrangement (50) has a cylinder-shaped antenna arrangement (50) for generating radio-frequency signals, which has a cylinder-shaped radio-frequency screen (40) around the antenna arrangement (50), wherein the antenna arrangement (50) comprises:
- multiple oscillating circuit antenna elements (60, 63, 66),
- multiple switching elements (59, 62, 65), wherein each oscillating circuit antenna element out of the multiple oscillating circuit antenna elements (60, 63, 66) is in each case assigned a switching element of the multiple switching elements (59, 62, 65), wherein a respective switching element is embodied to couple the assigned oscillating circuit antenna element operatively to the antenna arrangement (50) in dependence on a switching status of the switching element, and
- a control apparatus (14) embodied to carry out the following steps:
- measuring (101) symmetry information on the field distribution in the antenna arrangement (50), and
- automatically adjusting the multiple switching elements (59, 62, 65) using the measured symmetry information such that a symmetry of the field distribution in the interior of the antenna arrangement (50) is increased, **characterised in that** the symmetry of the field distribution is negatively impacted by a positional inaccuracy of the antenna arrangement (50) in the magnetic resonance system (10),
wherein the positional inaccuracy of the antenna arrangement (50) comprises a positional inaccuracy of an arrangement of the antenna arrangement (50) in respect of the radio-frequency screen (40), wherein the adjustment of the multiple switching elements (59, 62, 65) such that a symmetry of the field distribution in the interior of the antenna arrangement (50) is increased at least partially compensates the positional inaccuracy.

9. Magnetic resonance system according to claim 8, wherein the antenna arrangement (50) is embodied to carry out the method according to one of claims 2-7.

10. Magnetic resonance system according to claim 8 or claim 9, wherein the antenna arrangement (50) comprises two ferrules (52, 53), wherein each of the ferrules (52, 53) has in the circumferential direction multiple conductive surfaces (54, 55) that are insulated from one another, wherein one oscillating circuit antenna element out of the multiple oscillating circuit antenna elements (60, 63, 66) comprises a capacitor (60, 63), wherein the capacitor (60, 63) is arranged between two conductive surfaces (54, 55) of one of the ferrules (52, 53).

11. Magnetic resonance system according to claim 10, wherein the two conductive surfaces (54, 55) of the one of the ferrules (52, 53) have a capacitive coupling to one another, wherein the capacitor (60, 63) has a capacitance in the range of 1% to 20%, in particular in the range of 5% to 10%, of the capacitive coupling of the two conductive surfaces (54, 55).

12. Magnetic resonance system according to one of claims 8-11, wherein the antenna arrangement (50) comprises multiple longitudinal rods (51), wherein one oscillating circuit antenna element out of the multiple oscillating circuit antenna elements (60, 63, 66) comprises an inductive element (66), wherein the inductive element (66) is arranged parallel to one out of the multiple longitudinal rods (51) in the longitudinal direction.

13. Magnetic resonance system according to claim 12, wherein the one out of the multiple longitudinal rods (51) has an inductance, wherein the inductive element (66) has an inductance in the range of 1% to 20%, in particular in the range of 5% to 10%, of the inductance of the longitudinal rod (51) .

14. Computer program product, which comprises a program and can be loaded directly into a memory (21) of a programmable controller (20) of a magnetic resonance system (10) according to claim 8 to 13, with program means for carrying out all the steps of the method according to one of claims 1-7 when the program is executed in the controller (20) of the magnetic resonance system (10).

15. Electronically readable data medium with electronically readable control information stored thereupon, which is embodied to carry out the method according to one of claims 1-7 when the data medium is used in a controller (20) of a magnetic resonance system (10) according to one of claims 8 to 13.

## Revendications

1. Procédé de réglage d'une distribution de champ d'un dispositif d'antenne d'une installation de résonance magnétique,
dans lequel le dispositif (50) d'antenne est un dispositif (50) d'antenne de forme cylindrique pour la production de signaux de haute fréquence, qui a un écran (40) de haute fréquence de forme cylindrique tout autour du dispositif (50) d'antenne,
dans lequel le dispositif (10) d'antenne comprend plusieurs éléments (60, 63, 66) oscillants d'antenne et plusieurs éléments (59, 62, 65) de coupure, dans lequel, à chaque élément oscillant d'antenne parmi les plusieurs éléments (60, 63, 66) oscillants d'antenne est associé respectivement un élément de coupure parmi les plusieurs éléments (59, 62, 65) de coupure, dans lequel un élément respectif de coupure est conformé pour coupler, d'une manière fonctionnelle au dispositif (50) d'antenne l'élément oscillant d'antenne associé en fonction d'un état de commutation de l'élément de coupure, dans lequel le procédé comprend :
- mesure (101) d'une information de symétrie de la distribution de champ dans le dispositif (50) d'antenne, et
- réglage (101) automatique des plusieurs éléments (59, 62, 65) de coupure, à l'aide de l'information de symétrie mesurée, de manière à augmenter une symétrie de la distribution de champ à l'intérieur du dispositif (50) d'antenne, **caractérisé en ce que** l'on influence la symétrie de la distribution de champ par une imprécision de position du dispositif (50) d'antenne dans l'installation (10) de résonance magnétique, dans lequel l'imprécision de position du dispositif (50) d'antenne comprend une disposition du dispositif (50) d'antenne par rapport à l'écran (40) de haute fréquence, dans lequel le réglage des plusieurs éléments (59, 62, 65) de coupure, de manière à augmenter une symétrie de la répartition de champ à l'intérieur du dispositif (50) d'antenne, compense au moins en partie l'imprécision de position.

2. Procédé suivant la revendication 1, dans lequel la mesure de l'information de symétrie comprend une mesure d'une fréquence de résonance du dispositif (50) d'antenne, dans lequel on règle les plusieurs éléments (59, 62, 65) de coupure à l'aide de la fréquence de résonance, de manière à augmenter une symétrie de la distribution de champ à l'intérieur du dispositif (50) d'antenne.

3. Procédé suivant l'une des revendications précédentes, dans lequel la mesure de l'information de symétrie comprend une mesure d'un champ B1 dans le dispositif (50) d'antenne, dans lequel on règle les plusieurs éléments (59, 62, 65) de coupure à l'aide de la mesure de champ B1, de manière à augmenter une symétrie de la distribution de champ à l'intérieur du dispositif (50) d'antenne.

4. Procédé suivant l'une des revendications précédentes, dans lequel la mesure de l'information de symétrie comprend une mesure d'une distribution de courant dans le dispositif (50) d'antenne, dans lequel on règle les plusieurs éléments (59, 62, 65) de coupure à l'aide de la distribution de courant, de manière à augmenter une symétrie de la distribution de champ à l'intérieur du dispositif (50) d'antenne.

5. Procédé suivant l'une des revendications précédentes, dans lequel un élément oscillant d'antenne parmi les plusieurs éléments (60, 63, 66) oscillants d'antenne comprend un condensateur (60, 63), dans lequel le procédé comprend en outre :
- connexion du condensateur (60, 63) au dispositif (50) d'antenne au moyen de l'élément de coupure associé, afin de modifier une capacité du dispositif (50) d'antenne pour augmenter la symétrie de la distribution de champ dans le dispositif (50) d'antenne.

6. Procédé suivant l'une des revendications précédentes, dans lequel un élément oscillant d'antenne parmi les plusieurs éléments (60, 63, 66) oscillants d'antenne comprend un élément (66) inductif, dans lequel le procédé comprend en outre :
- connexion de l'élément (66) inductif au dispositif (50) d'antenne au moyen de l'élément de coupure associé pour modifier une inductance du dispositif (50) d'antenne, afin d'augmenter la symétrie de la distribution de champ dans le dispositif (50) d'antenne.

7. Procédé suivant l'une des revendications précédentes, dans lequel les plusieurs éléments (59, 62, 65) de coupure peuvent être fermés indépendamment les uns des autres.

8. Installation de résonance magnétique comprenant un dispositif (50) d'antenne, dans lequel le dispositif (50) d'antenne est un dispositif (50) d'antenne de forme cylindrique pour la production de signaux de haute fréquence, qui a un écran (40) de haute fréquence de forme cylindrique tout autour du dispositif (50) d'antenne, dans lequel le dispositif (50) d'antenne comprend :
- plusieurs éléments (60, 63, 66) oscillants d'antenne,
- plusieurs éléments (59, 62, 65) de coupure, dans lequel, à chaque élément oscillant d'antenne parmi les plusieurs éléments (60, 63, 66) oscillants d'antenne, est associé respectivement un élément de coupure parmi les plusieurs éléments (59, 62, 65) de coupure, dans lequel un élément respectif de coupure est conformé de manière à connecter fonctionnellement l'élément oscillant d'antenne associé au dispositif (50) d'antenne en fonction d'un état de commutation de l'élément de coupure, et
- une dispositif (14) de commande, qui est conformé pour effectuer les stades suivants :
- mesure (101) d'une information de symétrie de la distribution de champ dans le dispositif (50) d'antenne, et
- réglage automatique des plusieurs éléments (59, 62, 65) de coupure, à l'aide de l'information de symétrie mesurée, de manière à augmenter une symétrie de la distribution de champ à l'intérieur du dispositif (50) d'antenne, **caractérisé en ce que** l'on influence la symétrie de la distribution de champ par une imprécision de position du dispositif (50) d'antenne dans l'installation (10) de résonance magnétique, dans lequel l'imprécision de position du dispositif (50) d'antenne comprend une disposition du dispositif (50) d'antenne par rapport à l'écran (40) de haute fréquence, dans lequel le réglage des plusieurs éléments (59, 62, 65) de coupure, de manière à augmenter une symétrie de la répartition de champ à l'intérieur du dispositif (50) d'antenne, compense au moins en partie l'imprécision de position.

9. Installation de résonance magnétique suivant la revendication 8, dans laquelle l'agencement (50) d'antenne est conformé pour effectuer le procédé suivant l'une des revendications 2 à 7.

10. Installation de résonance magnétique suivant la revendication 8 ou la revendication 9, dans laquelle l'agencement (50) d'antenne comprend deux cerceaux (52, 53) d'extrémité, dans laquelle chaque cerceau (52, 53) d'extrémité a, dans la direction du pourtour, plusieurs surfaces (54, 55) conductrices isolées les unes des autres, dans laquelle un élément oscillant d'antenne parmi les plusieurs éléments (60, 63, 66) d'antenne comprend un condensateur (60, 63), dans laquelle le condensateur (60, 63) est disposé entre deux surfaces (54, 55) conductrices de l'un des cerceaux (52, 53) d'extrémité.

11. Installation de résonance magnétique suivant la revendication 10, dans laquelle les deux surfaces (54, 55) conductrices de l'un des cerceaux (52, 53) ont un couplage capacitif entre elles, dans laquelle le condensateur (60, 63) a une capacité dans la plage de 1 % à 20 %, notamment dans la plage de 5 % à 10 %, du couplage capacitif des deux surfaces (54, 55) conductrices.

12. Installation de résonance magnétique suivant l'une des revendications 8 à 11, dans laquelle le dispositif (50) d'antenne comprend plusieurs barreaux (51) longitudinaux, dans laquelle un élément oscillant d'antenne parmi les plusieurs éléments (60, 63, 66) oscillants d'antenne comprend un élément (66) inductif, dans laquelle l'élément (66) inductif est disposé parallèlement à l'un des barreaux (51) longitudinaux dans la direction longitudinale.

13. Installation de résonance magnétique suivant la revendication 12, dans laquelle le un des plusieurs barreaux (51) longitudinaux a une inductance, dans laquelle l'élément (66) inductif a une inductance dans la plage de 1 % à 20 %, en particulier dans la plage de 5 % à 10 %, de l'inductance du barreau (51) longitudinal.

14. Produit de programme d'ordinateur, qui comprend un programme et qui peut être chargé directement dans une mémoire (21) d'une commande (20) programmable d'une installation (10) de résonance magnétique suivant la revendication 8 à 13, comprenant des moyens de programme, pour effectuer tous les stades du procédé suivant l'une des revendications 1 à 7, lorsque le programme est réalisé dans la commande (20) de l'installation (10) de résonnance magnétique.

15. Support de données, déchiffrable électroniquement, sur lequel sont mises en mémoire des informations de commande pouvant être déchiffrées électroniquement, qui sont conformées de manière à effectuer, lors de l'utilisation du support de données dans une commande (20) d'une installation (10) de résonance magnétique suivant l'une des revendications 8 à 13, le procédé suivant l'une des revendications 1 à 7.
